Europäisches Patentamt

(19) European Patent Office

Office européen des brevets

(11) Publication number: **0 158 503**
**B1**

# (12) EUROPEAN PATENT SPECIFICATION

(45) Date of publication of patent specification: **10.08.88**

(21) Application number: **85302333.1**

(22) Date of filing: **03.04.85**

(51) Int. Cl.⁴: **C 07 C 5/333**, C 07 C 5/05, C 07 C 2/70, C 07 C 15/107

(54) **High selectivity process for dehydrogenation of paraffinic hydrocarbons.**

(30) Priority: **04.04.84 US 596772**
**04.04.84 US 596867**
**04.08.84 US 637234**

(43) Date of publication of application:
**16.10.85 Bulletin 85/42**

(45) Publication of the grant of the patent:
**10.08.88 Bulletin 88/32**

(84) Designated Contracting States:
**DE FR GB IT**

(56) References cited:
**US-A-3 413 373**
**US-A-3 432 567**
**US-A-3 459 822**
**US-A-3 484 498**

(73) Proprietor: **UOP Inc.**
**10 UOP Plaza Algonquin & Mt. Prospect Roads**
**Des Plaines Illinois 60016 (US)**

(72) Inventor: **Vora, Bipin Virpal**
**1686 Virginia Drive**
**Elk Grove Village Illinois 60007 (US)**

(74) Representative: **Hale, Stephen Geoffrey et al**
**J.Y. & G.W. Johnson Furnival House 14/18 High Holborn**
**London WC1V 6DE (GB)**

## Description

### FIELD OF THE INVENTION

The invention relates to the broad field of hydrocarbon processing. The invention may also be broadly classified as relating to a process for the production of olefinic hydrocarbons. More specifically, the invention relates to a hydrocarbon conversion process for the selective catalytic dehydrogenation of acyclic paraffinic hydrocarbons to produce monoolefinic hydrocarbons. The invention is directly concerned with the specific method used to perform a selective hydrogenation of diolefinic hydrocarbons within the dehydrogenation zone. The subject process may also include a step for the adsorptive separation of olefins and the recycling of unconverted paraffins. The olefinic hydrocarbon product may further be used in aromatic alkylation to produce alkylaromatic hydrocarbons. The selective hydrogenation converts diolefins present in the dehydrogenation reactor effluent stream to monoolefins.

### INFORMATION DISCLOSURE

Processes for the dehydrogenation of acyclic hydrocarbons are well known to those skilled in the hydrocarbon conversion arts. Such processes are operated commercially in petroleum refineries and petrochemical plants. A representative process flow diagram of a dehydrogenation process is provided in an article appearing at pages 86-88 of the January 26, 1970 issue of Chemical Engineering. The process is also described in U.S. Patents 3,484,498 issued to R. C. Berg and 3,494,971 issued to E. R. Fenske. The first reference illustrates the passage of a normal paraffin charge stream into a dehydrogenation reactor, with the effluent of this zone passing through a heat exchanger in which the vapor phase reactor effluent is partially condensed. The resultant mixed phase material is passed into a separating zone in which it is separated into a hydrogen-rich recycle stream and a liquid phase process stream. The liquid phase process stream is passed through a stripping zone which preferably comprises a trayed fractionation column. Hydrocarbons which remain after stripping off the light hydrocarbons are passed into an alkylation zone in admixture with benzene or other aromatic hydrocarbons. Hydrogen fluoride is the preferred alkylation catalyst. The effluent of the alkylation zone is passed into a fractionation system which produces several effluent streams including a product stream and a normal paraffin recycle stream which is directed into the dehydrogenation zone. The Fenske reference also illustrates the combination of a dehydrogenation zone with an alkylation zone, but supplies further details as to a preferred arrangement of the alkylation zone. These references also describe functional dehydrogenation catalysts and conditions.

U.S. Patent 3,696,160 issued to K. D. Chomyn is pertinent for its teaching that those skilled in the art of hydrocarbon processing are aware that it may be beneficial to selectively hydrogenate diolefins to monoolefins in certain hydrocarbon streams. This reference is directed to the selective conversion of propadiene and butadiene contaminants in propylene and butene charge stocks employed in alkylation processes for the production of aviation and motor fuel. In this alkylation process, a $C_3$-$C_4$ feed stream is converted to a high octane $C_7$-$C_8$ product. It is stated that a small diolefin content in the alkylation feed stream is undesirable because of increased acid consumption as a result of forming tarry acid-diolefin condensation products, which decreases the profitability of the process. The reference indicates that supported nickel and palladium catalysts are excellent hydrogenation catalysts in the diolefin conversion service, but that their tendency to deactivate in sulfur-containing feedstocks limits their utilization. The reference discloses the use of a sulfided nickel-tungsten catalyst.

U.S. Patent 3,655,621 issued to A. S. Kasperik et al illustrates a process for the selective hydrogenation of $C_4$ diolefins in an alkylation feed stream employing a catalyst comprising presulfided nickel supported on a refractory base. In U.S. Patent 3,234,298 issued to W. C. van Zijll Langhout et al, a process is disclosed for the selective hydrogenation of light, diene-containing cracked hydrocarbon oils. This process is employed to increase the stability of such materials as pyrolysis gasoline and kerosene obtained by severe thermal cracking operations. Such hydrogenation is desirable to reduce the gum-forming characteristics and other undesirable properties of these hydrocarbon mixtures. The process is described as being applicable to diene-containing hydrocarbons ranging from $C_3$ to $C_{18}$ in carbon number. The process employs a catalyst comprising sulfided nickel on alumina or sulfided molybdenum on alumina.

U.S. Patent 3,472,763 issued to J. Cosyns et al is pertinent for its description of a selective diolefin hydrogenation catalyst which comprises nickel supported on an alumina substrate having a number of specified characteristics and for its teaching of the utility of this catalyst. Specifically, it is taught that this catalyst may be employed for the conversion of all types of conjugated diolefins to monoolefins and in particular to the conversion of aliphatic conjugated diolefins having up to 15 carbon atoms per molecule to the corresponding monoolefins. The invention is also described as being useful in the selective hydrogenation of alpha alkenyl aromatic hydrocarbons to the corresponding alkylaromatic hydrocarbons. Another application of the process is the selective hydrogenation of gasolines containing diolefins and other gum-forming hydrocarbons.

The use of catalysts which comprise palladium supported on refractory material is described in U.S. Patents 3,662,015 issued to Y. Komatsu et al; 4,409,401 issued to T. P. Murtha; and 4,409,410 issued to J. Cosyns et al.

## BRIEF SUMMARY OF THE INVENTION

The invention provides an improved process for the production of acyclic olefinic hydrocarbons by eliminating or at least substantially reducing the concentration of diolefins in the dehydrogenation effluent stream. These improved results are obtained through the use of a selective hydrogenation reactor placed at a unique location within the overall dehydrogenation zone. The selective hydrogenation reactor is located between the product separator and the product stripper. The subject invention is therefore an integrated process rather than a separate post treatment step. This arrangement requires only a very minimal increase in the complexity and capital costs of the overall process to achieve a greatly significant improvement in operational performance.

One broad embodiment of the invention may be characterized as a process for the dehydrogenation of paraffinic hydrocarbons which comprises the steps of passing a feed stream comprising hydrogen and at least one $C_4$-plus paraffinic feed hydrocarbon through a dehydrogenation reaction zone maintained at dehydrogenation conditions and thereby producing a vapor phase dehydrogenation reactor effluent stream which comprises hydrogen, by-product light hydrocarbons having fewer carbon atoms per molecule than the feed hydrocarbon, $C_4$-plus paraffinic feed hydrocarbons, and $C_4$-plus mono- and diolefinic hydrocarbons; cooling and partially condensing the dehydrogenation reactor effluent stream and separating the dehydrogenation reactor effluent stream in a vapor-liquid separation zone into a vapor phase process stream which comprises hydrogen and a liquid phase process stream which comprises dissolved hydrogen, light hydrocarbons, $C_4$-plus paraffinic hydrocarbons, and $C_4$-plus mono- and diolefinic hydrocarbons; passing a hydrogen feed stream and the liquid phase process stream through a selective hydrogenation reaction zone which contains a selective hydrogenation catalyst and is maintained at diolefin selective hydrogenation conditions and forming a selective hydrogenation reaction zone effluent stream which comprises $C_4$-plus mono-olefinic hydrocarbons and is substantially free of $C_4$-plus diolefinic hydrocarbons; and passing the selective hydrogenation reaction zone effluent stream into a stripping column operated at conditions which result in the concentration of substantially all hydrogen and light hydrocarbon present in the hydrogenation zone effluent stream into a stripping column overhead stream, and producing a stripping column bottoms stream which comprises $C_4$-plus paraffinic hydrocarbons and $C_4$-plus monoolefinic hydrocarbons and which is removed from the process as a product stream.

It is also contemplated that the product stream may be passed into a separation zone in which olefinic hydrocarbons are concentrated into an olefin-rich product stream which is withdrawn, and also forming a separation zone effluent stream which is rich in the feed paraffins and which contains minor amounts of olefinic hydrocarbons; and passing at least a portion of the separation zone effluent stream into the dehydrogenation zone as a recycle stream.

Further, the broad concept of this invention may be employed in the production of alkylaromatic hydrocarbons by eliminating or at least substantially reducing the concentration in an alkylation zone effluent stream of undesirable by-products including biphenyl compounds, indanes, and tetralins. Besides increasing the purity of the desired monoalkylated benzenes, the subject process also increases the yield of these desired compounds. These improved results are obtained through the use of the selective hydrogenation reactor placed at a unique location within the overall dehydrogenation zone: between the product separation and the product stripper. The subject invention is therefore an integrated process rather than a separate feed pretreatment step. This arrangement requires only a very minimal increase in the complexity and capital costs of the process to achieve a greatly significant improvement in operational performance.

## DESCRIPTION OF THE DRAWINGS
### DRAWING I

Drawing I is a simplified process flow diagram of a preferred embodiment of the invention. Referring now to Drawing I, a paraffin feed stream comprising an admixture of $C_{10}$-$C_{15}$ normal paraffins enters the process through line 1. This feed stream is admixed with hydrogen from line 2 and passed into line 5. The mixture of paraffins and hydrogen flowing through line 5 is first heated in the indirect heat exchanger 6 and is then passed into a fired heater 7. The resultant vapor phase mixture of paraffins and hydrogen is passed through line 8 into a dehydrogenation reactor 9. Inside the reactor 9, the paraffins are contacted with a dehydrogenation catalyst at conditions which affect the conversion of a significant amount of the paraffins to the corresponding olefins.

There is thus produced a vapor-phase reactor effluent stream carried by line 10 which comprises a mixture of hydrogen, unconverted paraffins, $C_{10}$-$C_{15}$ monoolefins, a smaller amount of $C_{10}$-$C_{15}$ diolefins, and $C_1$-$C_9$ hydrocarbons produced as undesired by-products of the dehydrogenation reaction and by thermal cracking. This reactor effluent stream is first cooled by indirect heat exchange in the feed-product heat exchanger 6 and is then further cooled in the indirect heat exchange means 11. This cooling is sufficient to condense substantially all of the $C_{10}$-plus hydrocarbons into a liquid phase process stream which separates from the remaining vapor, which is rich in hydrogen. This mixed phase stream enters the vapor liquid separation vessel 12 wherein it is divided into a hydrogen-rich vapor phase stream removed through line 13 and a liquid phase process stream removed through line 14. The vapor phase stream is divided into a hydrogen product stream removed through lines 3 and 4 and a hydrogen recycle stream carried by line 2.

The liquid phase process stream removed from the bottom of the separator 12 contains unconverted $C_{10}$-$C_{15}$ paraffins, $C_{10}$-$C_{15}$ mono- and di-olefins, lighter hydrocarbons produced as by-products, and some dissolved hydrogen. A controlled volume of hydrogen from line 15 is admixed into the liquid process stream. It is then passed through line 16 into a selective hydrogenation reactor 17. In this reactor, the liquid phase hydrocarbons and hydrogen are contacted with a catalyst under conditions which promote the selective hydrogenation of diolefins to monoolefins. The liquid phase effluent of the selective hydrogenation reactor is then passed through line 18 to a stripper column 19. In this column, the light hydrocarbons produced in the dehydrogenation reactor and heater as by-products and any remaining unconsumed hydrogen are separated from the $C_{10}$-plus hydrocarbons and concentrated into a net overhead stream removed from the process through line 20. The remainder of the hydrocarbons entering the stripper are concentrated into a net bottoms stream carried by line 21, which is removed from the process as the product stream. This stream comprises an admixture of $C_{10}$-$C_{15}$ paraffins and monoolefins and has a greatly reduced concentration of diolefins compared to the dehydrogenation reactor effluent.

## DRAWING II

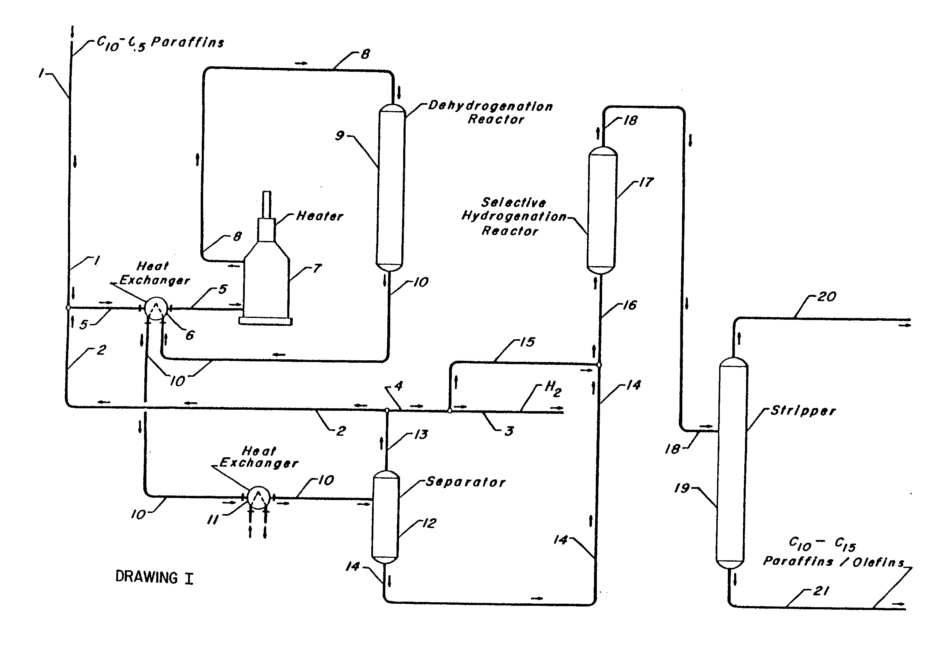

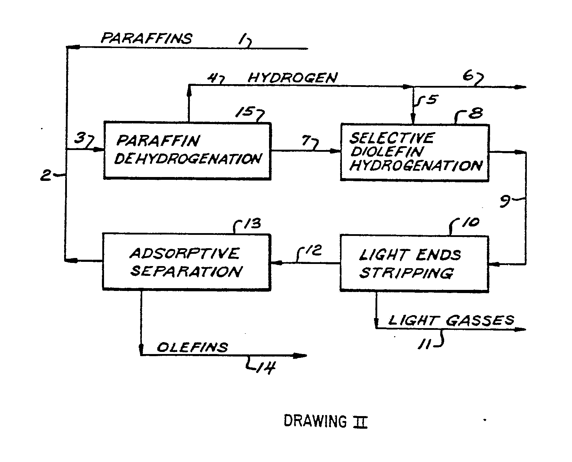

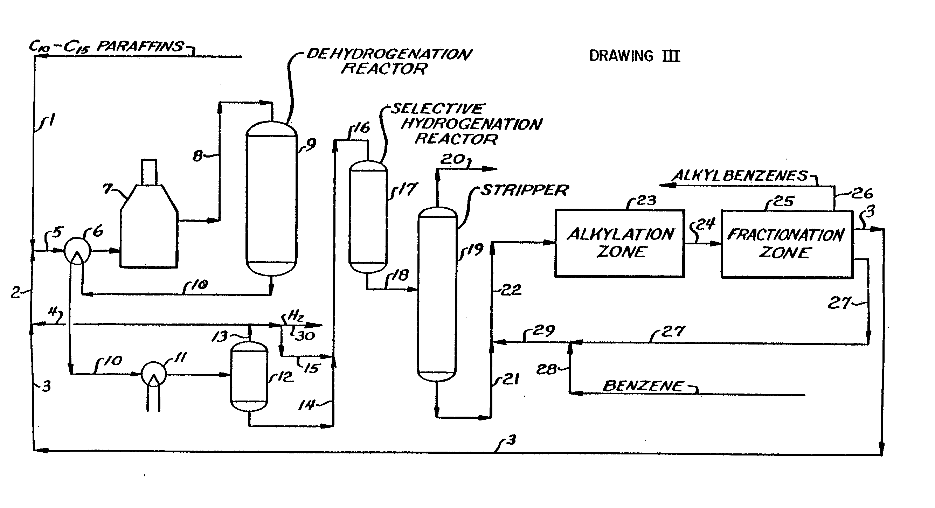

The drawing is a simplified flow diagram of a preferred embodiment of the invention. A feed stream comprising a mixture of $C_{10}$ to $C_{15}$ linear (straight chain) paraffins enters the process in line 1 and is admixed with a recycle stream from line 2, which comprises $C_{10}$ to $C_{15}$ paraffins and a minor amount of $C_{10}$ to $C_{15}$ monoolefins. The resultant reactor charge stream flows through line 3 into a paraffin dehydrogenation zone 15 wherein in the presence of a solid catalyst some of the paraffins are converted to monoolefins. Diolefins and light hydrocarbons are produced as by-products at a relatively small rate. A hydrogen-rich off gas stream comprising the net hydrogen of the reaction is vented off via lines 4 and 6. A liquid phase stream comprising condensed $C_{10}$-plus hydrocarbons, of which about 15 mole percent are olefins, and dissolved light hydrocarbons is passed through line 7 into a selective diolefin hydrogenation zone 8. Hydrogen from line 5 is added to ensure conversion of substantially all diolefins to monoolefins.

A hydrogenation zone effluent stream which is substantially free of diolefinic hydrocarbons is passed through line 9 into a light ends stripping zone 10 wherein such materials as hydrogen, methane, ethane, and in this case $C_9$-minus compounds are removed as the light gases removed in line 11. The remaining $C_{10}$ to $C_{15}$ paraffins and olefins pass through line 12 into an adsorptive separation zone 13 wherein over 90 mole percent of the olefins are concentrated into an olefin rich product stream removed from the process in line 14. The remaining paraffins and olefins are recycled through line 2 as the recycle stream, which is sometimes referred to as the separation zone raffinate stream.

## DRAWING III

The drawing is a simplified process flow diagram of a preferred embodiment of the invention. Referring now to the drawing, a paraffin feed stream comprising an admixture of $C_{10}$-$C_{15}$ normal paraffins enters the process through line 1. This feed stream is admixed with hydrogen and other normal paraffins from line 2 and passed through line 5. A mixture of paraffins and hydrogen flowing through line 5 is first heated in the indirect heat exchanger 6 and is then passed into a fired heater 7. The resultant vapor phase mixture of paraffins and hydrogen is passed through line 8 into a dehydrogenation reactor 9. Inside the reactor 9, the paraffins are contacted with a dehydrogenation catalyst at conditions which affect the conversion of a significant amount of the paraffins to the corresponding olefins. There is thus produced a reactor effluent stream carried by line 10 which comprises a mixture of hydrogen, unconverted paraffins, $C_{10}$-$C_{15}$ monoolefins, and a smaller amount of $C_{10}$-$C_{15}$ diolefins and $C_1$-$C_9$ hydrocarbons produced as undesired by-products of the dehydrogenation reaction. This reactor effluent stream is first cooled by indirect heat exchange in the feed-product heat exchanger 6 and is then further cooled in the indirect heat exchange means 11. This cooling is sufficient to condense substantially all of the $C_{10}$-plus hydrocarbons into a liquid phase process stream which separates from the remaining vapor, which is rich in hydrogen. This mixed phase stream enters the vapor-liquid separation vessel 12 wherein it is divided into a hydrogen-rich vapor phase stream removed through line 13 and a liquid phase process stream removed through line 14. The vapor phase stream is divided into a net hydrogen product stream removed through line 30 and a hydrogen recycle stream carried by line 4.

The liquid phase process stream removed from the bottom of the separator 12 contains unconverted $C_{10}$-$C_{15}$ paraffins, $C_{10}$-$C_{15}$ mono- and diolefins, lighter hydrocarbons produced as reaction by-products, and some dissolved hydrogen. A controlled volume of hydrogen from line 15 is admixed into the liquid process stream. It is then passed through line 16 into a selective hydrogenation reactor 17. In this reactor, the liquid phase hydrocarbons and hydrogen are contacted with a catalyst under conditions which promote the selective hydrogenation of diolefins to monoolefins. The liquid phase portion effluent of the selective hydrogenation reactor is then passed through line 18 to a stripper column 19. In this column, the light hydrocarbons produced in the dehydrogenation reactor as by-products and any remaining unconsumed hydrogen are separated from the $C_{10}$-plus hydrocarbons and concentrated into a net overhead stream removed from the process through line 20.

The remainder of the hydrocarbons entering the stripper are concentrated into a net bottoms stream carried by line 21. This stream comprises an admixture of $C_{10}$-$C_{15}$ paraffins and monoolefins and has a greatly reduced concentration of diolefins compared to the dehydrogenation reactor effluent. This admixture is combined with benzene from line 29 and passed into an alkylation zone 23 through line 22. In the alkylation zone, the benzene and olefinic hydrocarbons are admixed in the presence of an alkylation catalyst at alkylation-promoting conditions. The alkylation zone effluent stream carried by line 24 is passed into the fractionation zone 25. This stream comprises an admixture of unreacted benzene, $C_{10}$-$C_{15}$ paraffins, and the product alkylbenzenes. These compounds are separated into a paraffin recycle stream carried by line 3, a benzene recycle stream carried by line 27, and an alkylbenzene product stream carried by line 26. Benzene consumed in the process is charged through line 28.

## DETAILED DESCRIPTION

The production of acyclic olefinic hydrocarbons is a highly useful hydrocarbon conversion process. The product olefinic hydro-carbons find utility in the production of a wide variety of useful chemicals including synthetic lubricants, detergents, polymers, alcohols, plasticizers. Modern catalytic dehydrogenation processes provide a high degree of selectivity. However, they are still troubled by the production of light by-products, basically due to thermal cracking reactions and to undesired catalytic dehydrogenation side reactions. The by-products fall into two broad classes, light hydrocarbons formed by cracking reactions and diolefinic hydrocarbons having the same carbon number as the desired monoolefinic hydrocarbons. The production of diolefinic hydrocarbons is more troublesome, especially when the objective is to produce high purity monoolefinic hydrocarbons. The light by-products which result from cracking reactions can normally be readily removed from the olefin-containing product stream by a relatively easy fractional distillation step. In comparison, diolefinic hydrocarbons are normally much more difficult to remove from a product stream since their physical characteristics such as volatility are very close to those of the product monoolefins. Furthermore, the presence of diolefins in a dehydrogenation zone effluent is often undesirable because the diolefins react in downstream processes to form different compounds than the monoolefins.

It is therefore an objective of the subject invention to provide an improved process for the catalytic dehydrogenation of acyclic $C_4$-plus hydrocarbons. It is another objective of the subject invention to reduce the concentration of diolefinic hydrocarbons present in the product stream of a catalytic paraffin dehydrogenation process. It is a further objective to provide a method of selectively hydrogenating diolefins to monoolefins which has a very low capital and utilities cost.

Furthermore, the presence of diolefinic hydrocarbons in an integrated process, such as the subject process in which a paraffin-rich material may be recycled to the dehydrogenation zone, presents an additional problem. This problem is an increased rate of dehydrogenation catalyst deactivation compared to when feed boiling range hydrocarbons derived from the dehydrogenation reactor effluent are not returned to the dehydrogenation reactor. More specifically, the return of unsaturated hydrocarbons to the dehydrogenation zone inlet has been associated with catalyst lifes being reduced by 30 percent or more.

It is an objective of the subject invention to provide an improved process for converting linear parafins to linear monoolefins. It is another objective of the invention to increase the life of dehydrogenation catalyst employed in an integrated dehydrogenation-separation process for the production of linear monoolefins. It is a further objective to reduce the production of by-products such as light ends and diolefinic hydrocarbons and to thereby increase the overall yield of monoolefins in such a process.

Also, the production of alkylaromatic hydrocarbons by the sequential steps of paraffin dehydrogenation followed by an alkylation reaction is a well established commercial process. The product alkylaromatic hydrocarbons are tailored to a specific need through the choice of the feed paraffinic hydrocarbon and feed aromatic hydrocarbon. An example of this integrated process which has achieved widespread commercial success is the production of linear alkylbenzenes suitable for use in the production of detergent. These alkylated benzenes are often referred to in the art as detergent alkylate and the process is referred to as a detergent alkylation process. It is known to those skilled in the art that these processes currently generate a small but significant amount of undesired by-product hydrocarbons, which normally have a higher molecular weight and higher boiling point than the desired alkylbenzene. These undesired by-products are normally separated from the linear alkylbenzenes in the product recovery zone and concentrated into a stream referred to generically as heavy alkylate. Some of these heavy hydrocarbons, due to their greater solubility in HF acid, are also removed from the process as HF regenerator bottoms stream, sometimes known as polymer or acid soluble oils.

It is therefore a further objective of the subject invention to reduce the amunt of heavy alkylate and HF regenerator bottoms which is produced in an integrated detergent alkylation process. It is a further objective of the subject invention to improve the quality of the detergent alkylate produced in such an integrated process by reducing the production of undesired by-products which fall within the boiling point range of the desired alkylaromatic hydrocarbons.

The feed hydrocarbon charged to the subject process is an acyclic $C_4$-plus hydrocarbon. Prefer-

ably, the feed hydrocarbon is a normal paraffin. Paraffins which contain six or more carbon atoms per molecule are preferred over $C_4$ and $C_5$ paraffins. The upper limit on the carbon number of the charge stock is basically set by the volatility and processability of the charge stock in the dehydrogenation reactor. This upper limit is at about $C_{22}$ paraffins. The feed stream may be a high purity stream of a single paraffin or the feed stream may comprise a mixture of two or more paraffins having different carbon numbers. For instance, an admixture of $C_{10}$ to $C_{15}$ normal paraffins is often passed through a dehydrogenation zone to produce linear olefins which are consumed in the production of linear alkyl-benzenes suitable for use in the production of biodegradable detergents.

It has now been found that the objectives set forth above can be achieved through relatively simple modification of the dehydro genation unit. This modification performs a selective hydrogenation of diolefinic hydrocarbons produced in the dehydrogenation reactor. This selective hydrogenation converts at least a substantial amount of the diolefinic hydrocarbons to monoolefinic hydrocarbons, which are the desired product of the dehydrogenation unit. The concentration of undesired diolefinic hydrocarbons in the net effluent of the dehydrogenation section of the process is decreased. The subject process also provides a higher net yield of the desired monoolefins and a higher purity product. Perhaps most importantly, it has also been found that equipment required to perform the selective hydrogenation can be minimized by performing the hydrogenation step just downstream of the customary vapor-liquid or product separator of the dehydrogenation zone. This provides a low cost and facile method of performing the hydrogenation.

It is believed that heretofore attempts to improve the quality of an olefin-containing stream by selective hydrogenation have been in the form of separate processing operations which were not integrated into the dehydrogenation zone. That is, they appear to be described as separate operating steps which treat an olefinic feed stream charged to a selective hydrogenation process. They thus require separate process equipment such as heaters, control systems, product strippers, to deliver a product stream equivalent to that provided by the subject process.

The equipment used in the process is preferably configured substantially as shown in the drawings. In this arrangement, a fresh paraffinic hydrocarbon feed stream is combined with recycled hydrogen. This forms a reactant stream which is heated by indirect heat exchange and is then passed through a bed of a suitable catalyst maintained at the proper dehydrogenation conditions of temperature, pressure, etc. The effluent of this catalyst bed or reactor effluent stream is cooled and partially condensed. Part of the uncondensed material is employed as the hydrogen-rich recycle gas stream. The remainder of the uncondensed hydrogen-rich material is the net production of hydrogen which may be used in other applications such as desulfurization. As used herein, the term "rich" is intended to indicate a molar concentration of the indicated compound or class of compounds above 50%. The separation zone also produces a liquid stream referred to herein as the liquid phase process stream. This stream is basically an admixture of dehydrogenated and undehydrogenated acyclic hydrocarbons including diolefinic hydrocarbons. This liquid phase stream will also contain some dissolved hydrogen and light hydrocarbons produced by various cracking reactions which occur at the high temperatures employed in the dehydrogenation reactor.

In the subject process, the liquid phase process stream withdrawn from this separation zone is passed into a selective hydrogenation reaction zone. This zone contains a selective hydrogenation catalyst and is maintained at conditions necessary for selective hydrogenation of diolefins to monoolefins. The placement of the selective hydrogenation zone at this point makes it very simple and therefore very economical to perform the desired selective hydrogenation. One reason for this is that the reactants are in the desired liquid phase state as they leave the separation zone. A second reason is that the temperature of the liquid phase process stream as it leaves the separation zone will normally be within the desired operating range of the selective hydrogenation reaction zone.

This location for the hydrogenation zone is also preferred since it allows the effluent of the hydrogenation zone to be stripped of hydrogen in the same stripping column normally required for the removal of light ends from the liquid phase stream condensed out of the dehydrogenation reactor effluent. This stripping column is desired in a selective hydrogenation process to ensure that hydrogen does not enter downstream processing units. It is often highly undesired to charge hydrogen to a process unit. For instance, it is highly undesirable to admit hydrogen into an HF alkylation zone, since it would be necessary to vent this hydrogen from the alkylation zone and it would therefore be necessary to treat the vented hydrogen stream for the removal of vapor phase HF. Such treatment is costly and creates waste disposal problems. A separate but less important advantage to this process flow is that it allows at least partial utilization of the hydrogen dissolved in the liquid phase process stream in the hydrogenation step. This reduces the required rate of external hydrogen addition to the hydrogenation reactor. It thereby also increases the percentage of produced hydrogen which is available in the hydrogen-rich separator gas for removal from the process as a product stream. That is, the subject process flow at least partially consumes the dissolved hydrogen in the hydrogenation reaction rather than venting the hydrogen as part of a stripper overhead vapor.

The selective hydrogenation conditions employed in the hydrogenation zone are preferably similar to that maintained in the vapor-liquid separation zone of the prior art processes. More specifically, the minimum pressure should be sufficient to maintain the reactants as liquid phase hydrocarbons. A broad range of suitable operating pressures therefore extends from about 40 to about 1000 psig (276 to 6900 kPa gauge), with a pressure between about 50 and 300 psig (345 and 2070 kPa gauge) being preferred. A relatively moderate temperature between 25° and 250°C is preferred. More preferably, the hydrogenation zone is maintained at a temperature between 50° and 80°C. The liquid hourly space velocity of the reactants through the selective hydrogenation zone should be above 1.0. Preferably, it is above 5.0 and more preferably it is between 5.0 and 35 hr.$^{-1}$. The optimum set of conditions will of course vary depending on such factors as the composition of the feed stream and the activity and stability of the hydrogenation catalyst.

Another variable operating condition is the ratio of hydrogen to diolefinic hydrocarbons maintained within the selective hydrogenation zone. The amount of hydrogen required to achieve a certain conversion is believed dependent upon reactor temperature and the molecular weight of the feed hydrocarbons. Also, some catalysts, such as a palladium on alumina catalyst which was tested, require a higher hydrogen concentration to achieve the desired degree of hydrogenation. Therefore, with some catalysts, such as the palladium catalysts, it may be desired to operate with a hydrogen to diolefinic hydrocarbon mole ratio of between 2:1 and 5:1. With this catalyst, it was determined that hydrogen concentrations above this range resulted in the saturation of a significant amount of monoolefinic hydrocarbons. This of course is undesirable as it reduces the yield of the process. With the preferred feedstock and the preferred nickel sulfide catalyst, there should be less than 2.0 times the stoichiometric amount of hydrogen required for the selective hydrogenation of the diolefinic hydrocarbons which are present in the liquid phase process stream to monoolefinic hydrocarbons. Preferably, the mole ratio of hydrogen to diolefinic hydrocarbons in the material entering the selective hydrogenation zone is maintained between 1:1 and 1.8:1. In some instances, it may be desirable to operate with a less than stoichiometrically required amount of hydrogen, with mole ratios down to 0.75:1 being acceptable.

The selective hydrogenation zone preferably comprises a single fixed bed reactor containing a cylindrical bed of catalyst through which the reactants move in a vertical direction. It is preferred that the reactants flow upward through the reactor as this provides good mixing. The catalyst may be present as pellets, spheres, extrudates, irregular shaped granules. The prior art suggests the use of a number of metals on selective hydrogenation catalysts including tungsten, palladium, silver, molybdenum, and nickel. Of these metals, it is preferred that the active catalytic metal component present in the hydrogenation catalyst is either nickel or palladium, with nickel being especially preferred. When non-noble metals are employed, the catalyst should have a high concentration or loading of the active metal, with the metal component preferably comprising over 10 wt.% of the catalytic composite. More preferably, over 20 wt.% of the catalytic composite is metallic. It is very highly preferred that the selective hydrogenation catalyst also comprises a sulfur component. The preferred catalyst may therefore be described as a sulfided high nickel catalyst. The preparation of catalysts of this nature is described in U.S. Patent 3,919,341. The preferred selective hydrogenation catalyst has a lower sulfur concentration than the catalyst described in this reference, with sulfur levels between about 0.1 and 0.4 wt.% being preferred. The basic function of the sulfur component is believed to be the attenuation of the hydrogenation activity of the nickel. It is known in the art that carbon monoxide may be passed into a selective hydrogenation reactor for the purpose of moderating or attenuating the hydrogenation reaction. The use of carbon monoxide and other such moderators though not necessary may be employed.

The selective hydrogenation catalyst also comprises a support or carrier material which should be relatively inert and refractory to the conditions employed within the process. The support can be formed from a variety of porous materials including various clays, diatomaceous earth, aluminas, ceramics, attapulgus clay, and other synthetically prepared or naturally occurring silicates, kaolin, kieselguhr, titania, alumina, crystalline aluminosilicates, and admixtures of two or more of these materials. The especially preferred carrier material is an alumina. Of the aluminas, gamma-alumina is preferred. The carrier material or support may have an apparent bulk density of 0.3 to 0.8 g/cc, a surface area of 50 to 550 m$^2$/g, and a pore volume of between 0.l to 1.0 ml/g.

The effluent of the selective hydrogenation zone is a liquid phase stream similar in nature to the liquid phase process stream removed from the separator but having a reduced concentration of diolefinic hydrocarbons and a corresponding increase in the concentration of monoolefinic hydrocarbons. This effluent stream is passed into a stripping column designed and operated to remove overhead all compounds which are more volatile than the lightest hydrocarbon which it is desired to have present in the net effluent stream of the dehydrogenation process. These lighter materials will be concentrated into a net overhead stream which will comprise an admixture of hydrogen and light hydrocarbons. The purpose of the stripping operation is to prevent the entrance of volatile light materials into downstream processing zones where they would present certain operational problems. For example, the passage of light monoolefins into an alkylation zone would lead to the production of an increased amount of undesired side products through alkylation and polymerization reactions. The stripping column also serves to eliminate the light hydrocarbons from any recycle stream which returns paraffinic hydrocarbons to the dehydrogenation zone from the downstream processing units.

The composition of the dehydrogenation catalyst is not believed to materially affect the operation of the subject process provided this catalyst meets commercial standards for activity, stability, and selectivity. Dehydrogenation catalysts are described in U.S.-A-3,274,287; 3,315,007; 3,315,008; 3,745,112; and 4,430,517. These catalysts comprise a platinum group component supported on a porous carrier material. The preferred carrier material is a refractory inorganic oxide such as gamma-alumina. The preferred dehydrogenation catalysts contain on an elemental basis 0.01 to 2 wt.% platinum group component and 0.1 to 5 wt.% of an alkali or alkaline earth metal. Preferably, there is present 0-05 to 1 wt.% platinum group component and 0.25 to 3.5 wt.% of the alkali or alkaline earth component. The platinum group component may be chosen from the group consisting of platinum, palladium, rhodium, ruthenium, osmium, and iridium, but platinum is highly preferred. The alkali or alkaline earth component may be selected from the group consisting of the alkali metals-cesium, rubidium, potassium, sodium, and lithium; and the alkaline earth metals-calcium, strontium, barium, and magnesium. This component is preferably either lithium or potassium, with lithium being especially preferred. Another example of a suitable dehydrogenation catalyst is a catalyst which in addition to the previously described platinum and alkali or alkaline earth metal components contains a tin component. This catalytic composite would contain from 0.1 to 1 wt.% tin. Yet another catalytic composite which should be highly suited for use in the subject process comprises an indium component in addition to the platinum, tin, and alkali or alkaline earth components. The indium component may be present on an elemental basis equal to 0.1 to 1 wt.% of the final composite. It is also known in the art that some catalytic composites of this nature may benefit from the presence of a small amount of a halogen component, with chlorine being the normally preferred halogen. Typical halogen concentrations in the final catalytic composite range from 0.1 to 1.5 wt.%. A halogen component is not desired in all situations. These catalytic composites are known to those skilled in the art and are described in the available references.

A preferred embodiment of the invention may accordingly be characterized as a process for the dehydrogenation of paraffinic hydrocarbons which comprises the steps of passing hydrogen and a paraffin feed stream which comprises at least one $C_{10}$-plus linear paraffinic hydrocarbon through a catalytic dehydrogenation reaction zone and forming a vapor phase dehydrogenation reaction zone effluent stream which comprises a mixture of hydrogen, $C_8$-minus dehydrogenation reaction by-product hydrocarbons, $C_{10}$-plus mono- and diolefinic linear hydrocarbons, and $C_{10}$-plus linear paraffins; separating hydrogen from the dehydrogenation reaction zone effluent stream by partially condensing the dehydrogenation reaction zone effluent stream and separating the resultant two-phase admixture in a vapor-liquid separation zone and forming a vapor phase stream which is rich in hydrogen and a liquid phase process stream comprising $C_8$-minus by-product hydrocarbons, $C_{10}$-plus linear paraffins, dissolved hydrogen, and mono- and diolefinic $C_{10}$-plus linear hydrocarbons; passing a hydrogen feed stream and the liquid phase process stream through a selective hydrogenation zone maintained at diolefin selective hydrogenation conditions and in which the liquid phase process stream is contacted with a solid selective hydrogenation catalyst and thereby forming a hydrogenation zone effluent stream which contains less than 0.4 mole percent $C_{10}$-plus diolefinic hydrocarbons; removing substantially all dissolved hydrogen and $C_8$-minus hydrocarbons from the hydrogenation zone effluent stream by passing the hydrogenation zone effluent stream into a light ends stripping column, and producing a stripping column bottoms stream which comprises a mixture of $C_{10}$-plus monoolefinic linear hydrocarbons and $C_{10}$-plus linear paraffins and which is removed from the process as a net product stream.

The net product of the process, the bottoms stream of the stripping column, can be passed into a number of downstream processing units or it can be withdrawn as a finished product. For instance, the product stream may be passed into alkylation zones wherein the olefinic hydrocarbons can be reacted with aromatic hydrocarbons or into esterification zones as in the production of plasticizers. The product stream may also be passed into an oligomerization zone or a hydration zone. Another possibility is that the net process effluent stream may be charged to a separation zone which separates the monoolefins from the unconverted paraffins.

This separation could be performed by fractional distillation on a single carbon number effluent stream but would be quite a difficult and expensive fractionation. Sorptive-type separations which employ selective solid adsorbents. known in the art, are preferred for this type of separation. A broad carbon number range olefin-paraffin mixture can be charged to such a process and efficiently separated into a high purity olefin stream and a paraffin stream. The paraffin stream may then be recycled to the dehydrogenation zone.

Where the net process effluent stream is charged to a separation zone which separates the monoolefins from unconverted paraffins, the paraffins may be recycled to the dehydrogenation zone. Selective hydrogenation will greatly reduce or eliminate increased dehydrogenation catalyst deactivation observed when hydrocarbons are recycled from the separation zone. This is based on the concept that it is the presence of diolefinic hydrocarbons at the inlet of the catalyst bed which promotes coke deposition and catalyst deactivation. In a nonrecycle or olefin-free recycle dehydrogenation process, diolefinic hydrocarbons are rapidly removed from the catalyst bed due to the high space velocities normally employed in dehydrogenation reactors. Further, it is believed that it is necessary to first dehydrogenate a paraffin to a monoolefin before any diolefins are produced. The production of diolefins is, therefore, greatest at the near equilibrium conditions at the exit of a nonrecycle reactor, and most of the catalyst bed is accordingly spared severe deactivation by coke formation. Coke formation is believed to follow the production of polymeric or cyclic "coke precursors" which are derived from the diolefins. The rapid removal of diolefins from the reaction zone coupled with their formation in the later stages of the dehydrogenation reaction tends to minimize coke formation on the catalyst. In comparison the recycling of diolefins to the reactor tends to promote coke deposition and catalyst deactivation. The diolefins are then present in the entire catalyst bed and the whole bed is subjected to deactivation. The subject invention greatly reduces or eliminates the presence of diolefins in the charge stream of the reactor. It is believed this will be sufficient to significantly lengthen dehydrogenation catalyst life even though the charge stream still contains monoolefins.

The effect of the composition of a paraffinic recycle stream on the life of a dehydrogenation catalyst may be observed by a comparison of two common processes, both of which employ a dehydrogenation zone to produce linear olefins. In each case, a liquid phase stream containing the olefins and remaining paraffins is produced in the dehydrogenation zone. In a process flow in which this olefin-containing stream is passed into a reaction zone in which the monoolefins, and diolefins, are totally consumed and the remaining paraffins are recycled to the dehydrogenation zone, the preferred dehydrogenation catalyst would be expected to process about 220 barrels of paraffins before it is necessary to replace or regenerate the catalyst. An example of this process is the production of alkylbenzenes by the HF-catalyzed alkylation of benzene as described in U.S. Patents 3,484,498; 3,494,971; 3,950,448; and 4,225,737. The HF is a very effective catalyst. All of the olefinic compounds not consumed in the alkylation reaction are consumed in oligomerization reactions. The paraffin recycle stream, therefore, is essentially free of all olefinic hydrocarbons. In comparison, the recycle stream from an absorptive separation in a process similar to that shown in previously referred to U.S. Patent 3,617,504 will contain olefins and diolefins. Table VI of this reference gives specific amounts of paraffins, olefins, and diolefins in the recycle stream to the dehydrogenation zone. In a process such as this, it would be expected that the dehydrogenation catalyst would process about 150 barrels of paraffins per pound of catalyst before it is necessary to replace the catalyst. This is based on the same reactor conditions and same feedstock as the previously given catalyst life. It may thus be seen that rather severe premature catalyst deactivation occurs in the latter case due to the presence of the various olefinic hydrocarbons in the recycle stream. As previously described it is believed that the majority of this increased deactivation is caused by the presence of diolefins in the cycle stream.

The type of separation zone used to recover the product olefins from the paraffin-olefin mixture is not a limiting characteristic of the subject process. Separatory techniques based upon liquid-liquid extraction or chemical binding can, therefore, be employed if they meet commercial standards of cost, effectiveness, and workability. It is greatly preferred that an absorptive type separation is employed, with the paraffin-olefin mixture being passed through a bed of a solid adsorbent which selectively collects either the olefins or the paraffins from the flowing stream. The selective retention of the olefins is preferred as there is a smaller quantity of olefins. This could be performed in a simple swing bed system with one or more beds being used to collect olefins while previously used beds are being regenerated as by the use of a desorbent, a temperature increase, a pressure decrease, or a combination of these commonly employed regeneration techniques. Another type of adsorptive separation process which may be employed is described in U.S.-A-4,402,832. This process simulates continuous cocurrent movement of the adsorbent relative to the fluid flow.

A preferred configuration of the adsorptive separation zone is described in U.S.-A-3,239,455; 3,617,504; and 4,133,842. The use of this separatory technique to separate olefinic hydrocarbons from a paraffin-olefin mixture is the subject of U.S.-A-3,510,423; 3,720,604; 3,723,302; and 3,755,153. These references describe operating conditions and methods and suitable adsorbents. They also describe in some detail the use of a preferred technique for simulating countercurrent flow of the feed hydrocarbons and the adsorbent. A variation in the equipment used to perform this process is the subject of U.S.-A-4,434,051. Adsorptive separations can be performed using either vapor phase or liquid phase conditions within the adsorption zone. The use of liquid phase methods is preferred as it allows operation at lower temperature, which minimizes any polymerization of olefins. Operating conditions for the adsorbent chambers can include a temperature of from 25° to 225°C and a pressure of from atmospheric to 750 psig (0 to 5200 kPa gauge).

A sorptive separation step can be practiced using any type of commercially operable and practical selective adsorbent. The adsorbent may, therefore, be a naturally occurring substance or a man-made material and may be in the form of extrudates, pellets, spheres. The adsorbent can be formed from charcoal, alumina, silica, or various clays, and mixtures of these materials. The preferred adsorbent comprises a selective zeolite commonly referred to as a molecular sieve. The preferred zeolites comprise synthetic crystalline aluminosilicates. Since the pure zeolites are relatively soft and powdery, the commercially used molecular sieves comprise a binder such as clay or alumina to produce a stronger and more attrition-resistant adsorbent particle. The adsorbent particles preferably have a size range of about 20 to about 40 mesh. The adsorbents which can be used in the process include the Type X or Type Y structured crystalline aluminosilicates or the naturally occurring faujasite species. The Type X zeolite is described in U.S. Patent 2,882,244 while the Type Y zeolite is described in U.S. Patent 3,130,007. The adsorbents as described above can contain cations selected from the group consisting of alkali metals (Group I-A), the alkali-earth metals (Group II-A), the coinage metals (Group I-B), or the Group II-B metals. Preferred metals selected from the aforementioned group include lithium, sodium, potassium, magnesium, calcium, strontium, barium, copper, silver, gold, zinc, cadmium, and mercury. Additionally, combinations of the above-mentioned metals may be included to enhance the adsorbent's selectivity for the olefins and to help reduce the harmful effects of side reactions including polymerization.

It is preferred to remove olefins from the adsorbent through the use of a liquid phase desorbent which is passed through the adsorbent bed. The desorbents which can be used in this process include olefinic-type hydrocarbons which boil at temperatures sufficiently different than the boiling tempeature of the feedstock. Both branched chain or straight chain monoolefins can be used as desorbents. Additionally, aromatic-type hydrocarbons may be used as desorbents. In some instances, it will be advantageous to employ desorbents which contain a mixture of normal olefins or isoolefins and, normal or isoparaffins or paraffins or aromatics. Typical desorbents which can be used for a feedstock containing $C_{10}$ to $C_{14}$ monoolefins and paraffins is a desorbent mixture comprising about 80 volume percent octene-1 and 20 volume percent isooctane. In most instances, it is preferred to use a lower molecular weight desorbent mixture as compared to the feedstock. An example of a desorbent which can be used when $C_6$ to $C_9$ feedstock being separated is a desorbent containing about 80% of a straight chain butylene and about 20 volume percent of normal butane.

Most separation methods do not perform perfect separations. In large scale commercial processes, it often becomes prohibitively costly to perform a complete recovery or separation of hydrocarbons. This is also true in the case of absorptive recovery of olefins from the paraffin-olefin mixture. Recoveries of olefinic hydrocarbons can exceed 99 mole percent with the preferred simulated moving bed system, but the purity of the olefin product suffers as the recovery is increased. For this reason, most commercial process units are designed and operated to recover from 90 to 95 mole percent of the olefins per pass. Therefore, 5 to 10 mole percent of the original olefinic hydrocarbons will remain in the paraffinic recycle or raffinate stream which is passed into the dehydrogenation zone. This recycle stream will contain from 0.4 to 1.9 mole percent monoolefins in the subject process. This olefin concentration is significant due to its effects on the required operation of the dehydrogenation zone, as previously discussed, in comparison to processes which recycle an olefin-free hydrocarbon stream to the dehydrogenation zone.

A preferred embodiment of the invention may accordingly be characterized as a process for the production of $C_6$-plus linear monoolefins which comprises the steps of passing a feed stream comprising at least two $C_6$-plus feed paraffins and a hereinafter characterized recycle stream into a catalytic dehydrogenation zone operated at dehydrogenation conditions and producing a hydrogen-rich gas stream and a liquid phase dehydrogenation zone effluent stream which comprises dehydrogenation by-product light hydrocarbons, the two feed paraffins, and corresponding monoolefinic and diolefinic hydrocarbons; passing the dehydrogenation zone effluent stream through a catalytic selective hydrogenation zone operated at conditions effective to selectively convert diolefinic hydrocarbons to monoolefinic hydrocarbons without saturating monoolefinic hydrocarbons and thereby forming a hydrogenation zone effluent stream which comprises the dehydrogenation by product light hydrocarbons, the two feed paraffins and corresponding monoolefinic hydrocarbons and which has a lower concentration of diolefinic hydrocarbons than the dehydrogenation zone effluent stream; removing dissolved hydrogen and by-product light hydrocarbons from the hydrogenation zone effluent stream in a fractionation zone; contacting the hydrogenation zone effluent stream with a solid adsorbent in an absorptive separation zone and separating the hydrogenation zone effluent stream into a product stream, which is rich in the olefinic hydrcarbons and is withdrawn from the process, and a separation zone effluent stream which is rich in the feed paraffins and which also contains minor amounts of olefinic hydrocarbons; and passing at least a portion of the separation zone effluent stream into the dehydrogenation zone as the previously referred to recycle stream. This recycle stream is preferably passed directly into the dehydrogenation zone as shown in the drawing. However, it must be realized that in commercial scale operations process streams may be routed through storage facilities which serve to buffer flow rate differences between different process units. The recycle stream and other streams may therefore flow through tankage or surge drums within the integrated process. Other fractionation steps can also be employed within the overall process to remove undesired compounds, etc.

When the product stream is passed into an alkylation zone, it is preferred that the feed paraffinic hydrocarbon is a $C_{10}$ to $C_{15}$ paraffin because a better quality detergent precursor normally results from the use of olefinic hydrocarbons having from about 10 to 15 carbon atoms per molecule. The aromatic hydrocarbon which is alkylated in the subject process is preferably benzene, but a higher molecular weight aromatic hydrocarbon may also be charged to the process. The feed aromatic hydrocarbon may therefore be toluene, a xylene, ethylbenzene, phenol, naphthalene.

In the subject process, the feed paraffinic hydrocarbons are first converted to olefinic hydrocarbons in a dehydrogenation zone. The unseparated paraffin/olefin mixture produced as the effluent of the dehydrogenation zone is then passed into the alkylation zone as the olefin-containing feed stream. This is basically because of the high cost of separating olefins and paraffins of the same carbon number, but the presence of the paraffins can also be beneficial as by decreasing the overall olefin concentration in the alkylation reactor and acting as a heat sink for the heat of reaction. The olefin-containing feed stream charged to the alkylation zone may therefore contain from 30 to 92 mole percent straight chain paraffins having the same number of carbon atoms per molecule as the olefinic hydrocarbon. These relatively non-reactive paraffins pass through the alkylation zone in various hydrocarbon phase streams and are eventually separated from the alkylate by fractionation and then recycled to the dehydrogenation zone.

It has no been found that the objectives set forth above can be achieved through modification of the dehydrogenation section of the integrated process. This modification comprises the selective hydrogenation of diolefinic hydrocarbons produced in the dehydrogenation reactor. This selective hydrogenation converts at least a substantial amount of the diolefinic hydrocarbons to monoolefinic hydrocarbons, which are the desired product of the dehydrogenation section. At the same time, the concentration of undesired diolefinic hydrocarbons in the net effluent of the dehydrogenation section of the process is decreased. The lower concentration of diolefinic hydrocarbons in the alkylation zone results in a reduced production of by-products including oligomers and biphenyl hydrocarbons. It also is expected to produce a decrease in the consumption of the preferred HF alkylation catalyst. It has also been found that equipment required to perform the selective hydrogenation can be minimized by performing the hydrogenation step just downstream of the customary vapor-liquid or product separator of the dehydrogenation zone. This provides a low cost and facile method of performing the hydrogenation.

It is believed that heretofore attempts to improve the selectivity or product quality of detergent alkylation processes have concentrated on improvements within the alkylation zone itself. These improvements were in such areas as promoting a better or more uniform admixture and contacting of the various hydrocarbon phases with the alkylation catalyst, the optimization of such alkylation conditions as temperature, pressure, and concentrations, the use of additives and modifiers within the alkylation catalyst.

A preferred embodiment of the invention may accordingly be characterized as a process for the dehydrogenation of paraffinic hydrocarbons which comprises the steps of passing a paraffin feed stream which comprises at least one $C_{10}$-plus linear paraffinic hydrocarbon through a dehydrogenation reaction zone and forming a vapor phase dehydrogenation reaction zone effluent stream which comprises a mixture of hydrogen, monoand diolefinic $C_{10}$-plus linear hydrocarbons, and $C_{10}$-plus linear paraffins; separating hydrogen from the dehydrogenation reaction zone effluent stream by partially condensing the dehydrogenation reaction zone effluent stream and separating the resultant two-phase admixture in a vapor-liquid separation zone and forming a vapor phase stream which is rich in hydrogen and a liquid phase process stream comprising $C_{10}$-plus linear paraffins, dissolved hydrogen, and mono- and diolefinic $C_{10}$-plus linear hydrocarbons; passing the liquid phase process stream through a selective hydrogenation zone maintained at diolefin selective hydrogenation conditions and in which the liquid phase process stream is contacted with a solid selective hydrogenation catalyst and thereby forming a hydrogenation zone effluent stream which contains less than 0.4 mole percent $C_{10}$-plus diolefinic hydrocarbons; removing substantially all free hydrogen and $C_6$-minus hydrocarbons from the hydrogenation zone effluent stream by passing the hydrogenation zone effluent stream into a light ends stripping column, and producing a stripping column bottoms stream which comprises a mixture of $C_{10}$-plus monoolefinic linear hydrocarbons and $C_{10}$-plus linear paraffins; contacting the stripping column bottoms stream with an alkylation catalyst and with benzene within an alkylation zone maintained at alkylation-promoting conditions, and producing an alkylation zone effluent stream which comprises $C_{10}$-plus linear paraffins and alkylbenzenes, and recovering the alkylbenzenes from the alkylation zone effluent stream.

The net effluent of the dehydrogenation section is passed into an alkylation section, which comprises an alkylation zone and a fractionation or alkylate recovery zone. The alkylation zone can have a number of different configurations depending on the catalyst and reactor vessels which are employed. A solid alkylation catalyst could be employed in the alkylation zone. For example, one current trend in heterogeneous alkylation catalysts is the use of a zeolitic catalyst as described in U.S. Patents 3,751,506; 4,387,259; and 4,409,412. The use of a homogeneous mineral acid catalyst is however greatly preferred, with liquid phase HF being especially preferred.

Chemical reactions which involve olefinic hydrocarbons and are catalyzed by hydrogen fluoride usually proceed at a very fast rate. To reduce the amount of olefin polymerization and to promote the production of a monoalkylated aromatic product, the reactants are normally subjected to vigorous mixing and agitation at the point of initial contact of the olefinic hydrocarbons and the liquid phase hydrogen fluoride. The desired result is a uniform dispersion and intimate contacting of the hydrocarbon and hydrogen fluoride phases and the avoidance of localized high temperatures or localized high concentrations of either the olefinic hydrocarbon or the hydrogen fluoride. The initial contacting of the reactants and the catalyst has been done in a number of different ways. For instance, the olefinic hydrocarbons have been sprayed into a mixture of hydrogen fluoride and hydrocarbons through nozzles, and mixtures of the reactants have been released into eductors as high velocity streams which cause the eduction and admixture of the hydrogen fluoride. U.S. Patent 4,134,734 describes a unitary reactor for the production of detergent alkylate. U.S. Patent 4,072,730 describes a process for producing detergent alkylate in which a centrifugal pump is utilized as the first reaction zone due to the intense agitation which occurs within the pump.

The alkylation zone preferably has an overall arrangement similar to that shown in previously referred to U.S. Patent 3,494,971. An improvement to this is shown in U.S. Patent 4,225,737. In this arrangement, the two feed hydrocarbon streams and liquid phase HF are charged to a reactor. The effluent of this reactor is passed into a first settling zone and separated into HF and hydrocarbon phases. The settling zones are preferably elongated horizontal vessels. The HF is withdrawn and divided into a first portion passed into the HF regeneration column for regeneration and a second portion returned to the reactor. The hydrocarbon phase is withdrawn from the first settling zone and charged to a contactor, which is sometimes referred to as the second "reactor", as the only hydrocarbon charged to the contactor. The HF charged to the contactor is a mixture of newly regenerated HF and HF withdrawn from a second settling zone, which receives the total effluent of the contactor. A portion of the HF withdrawn from the second settling zone is charged to the reactor to replace the HF withdrawn for regeneration. The hydrocarbon phase which is withdrawn from the second settling zone may be withdrawn as an alkylation zone effluent stream but is preferably passed into a stripping column in which dissolved HF is removed overhead and some of the feed aromatic hydrocarbon is also recovered. The net bottoms of this HF stripping column is charged to the fractionation or other product recovery zone employed in the process. It may be desirable to depart from these preferences and employ more than one reactor.

The alkylation reactor and the contactor are maintained at alkylation-promoting conditions. As used herein, the term alkylation-promoting conditions is intended to include a pressure sufficient to maintain the reactants and HF in a liquid phase. A general range of operating pressures is from 2 to 41 atmospheres absolute (200 to 4100 kPa gauge). The temperature range covered by this set of conditions is from -20° to 95°C, but the reaction is preferably con ducted at a temperature of from 15° to 50°C. The volumetric ratio of HF to the total amount of hydrocarbons entering the reactor should be maintained within the broad range of from 0.2:1 to 10:1. A preferred range for this ratio is from 1:1 to 2.5:1. To lessen the production of polyalkylated benzenes and to reduce the amount of olefin polymerization in the reactor, the mole ratio of benzene to the monoolefin at the point of initial olefin-acid contact is maintained above 1:1, but preferably below 10:1. A range of typical commercial ratios is from 3:1 to 8:1.

The conditions maintained within the contactor are similar to the conditions maintained in the reactor, but some adjustment is required. For instance, since essentially all of the olefin is preferably consumed in the reactor, the hydrocarbon stream fed to the contactor is substantially free of olefins. There is therefore no benzene to olefin ratio to be specified. The same pressure range may be used in the contactor as in the reactor, but a higher temperature is preferred. This higher temperature should be at least 6 to 10 Centigrade degrees above that used in the reactor. All temperatures specified herein are intended to refer to the average temperature of the liquid stream entering the respective zone.

The HF/hydrocarbon ratio maintained in the contactor will normally be slightly lower, and a typical ratio is about 1:1. The purity of acid used in the contactor will, however, be higher. This is preferred because of the greater effectiveness of higher purity acid for the treatment of the alkylate. This treatment consists of the defluorination of the alkylate product and the extraction of naphthalenes and anthracenes. A higher acid purity is obtained by admixing the newly regenerated acid into the alkylate-containing hydrocarbon stream entering the contactor. The recycle acid for use in the reactor is withdrawn from the second settling zone and therefore contains a higher concentration of high molecular weight hydrocarbonaceous impurities. The acid used in the reactor preferably contains about 85-92 wt.% HF and will typically be about 90 wt.% HF. The acid used in the contactor preferably contains more than 90 wt.% HF and is typically about 93-94 wt.% HF.

The effluent streams leaving the reactor and the contactor will typically be an intimate admixture of liquid phase hydrocarbons and liquid phase hydrogen fluoride. They may be in the form of a true emulsion. A considerable residence time is normally required to separate these two liquid phases, and the effluent streams are therefore passed into quiescent settling zones. The two settling zones will normally be maintained at a temperature which is set by the entering HFhydrocarbon mixtures withdrawn from the respective upstream vessels. They will therefore be at substantially the same temperature as the immediately upstream reaction or contacting zone. The same is also normally true for the pressures used in the settling zones after adjustment for any pressure change due to liquid flow and elevation differences. The settling zones may however be downstream of control valves and therefore operated at a somewhat reduced pressure. This reduced pressure, however, must be superatmospheric and sufficient to maintain liquid phase conditions. A residence time for both the acid and hydrocarbon phases in the settling zones should be in excess of 90 seconds but less than 30 minutes.

The hydrocarbonaceous phase removed from the second settling zone is preferably passed into a fractionation column commonly referred to as the HF stripping column. This column derives its name from its basic function in the prior art of preventing the passage of HF into the downstream fractionation zone. Representative conditions for the operation of the HF stripping column include an overhead vapor temperature of about 250°F (120°C) at a pressure of approximately 36 psig (250 kPa gauge). There is normally no external reflux to this column. The overhead vapor stream of the HF stripping column is normally competely condensed by cooling it to about 100°F or less and is then decanted and recirculated as described above. The entire hydrocarbonaceous effluent of the second settling zone is normally passed onto the top tray of this column. The net bottoms stream of this column contains the product alkylate.

Fractionation systems and conditions suitable for use as an effective separation zone to recover the product alkylate from the bottoms stream of the HF stripping column are described in U.S. Patents 3,950,448; 4,237,327; and 4,237,328. For instance, the bottoms stream of the HF stripping column is preferably passed into a second fractionation column referred to as a benzene column. The benzene column is operated under conditions effective to cause the division of the entering hydrocarbons into a high purity benzene stream which is removed as the overhead liquid and a bottoms stream containing the alkylate product. This bottoms stream is passed into a third fractionation column referred to as a paraffin column. The non-reactive paraffins are removed as an overhead liquid stream. The bottoms stream of the third fractionation column comprises the product alkylate and any higher molecular weight side product hydrocarbons formed in the reaction zone. This bottoms stream is passed into a fourth fractionation column which produces a high purity overhead stream containing the detergent alkylate. A bottoms stream comprising polymerized olefins and polyalkylated benzenes (heavy alkylate) is removed from the fourth column for disposal. The third and the fourth fractionation columns are normally operated at a subatmospheric pressure. An alternative method of performing this separation is disclosed in previously cited U.S. Patent 3,950,448. In this arrangement, the bottoms stream of the HF stripping column is passed into a column referred to as a paraffin column. All of the feed aromatic hydrocarbon is withdrawn from the HF stripping column in an overhead stream or as a liquid stream removed below a contact condenser located in the top of the column. The net bottoms stream of the HF stripping column is therefore devoid of the feed aromatic hydrocarbon. This bottoms stream is then separated in the same manner as set out above.

## Claims

1. A process for the dehydrogenation of a paraffinic hydrocarbon which comprises the steps of:

(a) passing a feed stream (8) comprising hydrogen and at least one $C_4$-plus paraffinic feed hydrocarbon through a dehydrogenation reaction zone (9) maintained at dehydrogenation conditions and thereby producing a vapor phase dehydrogenation reactor effluent stream (10) which comprises hydrogen, by-product light hydrocarbon(s) having fewer carbon atoms per molecule than the feed hydrocarbon, $C_4$-plus paraffinic feed hydrocarbon(s), and $C_4$-plus mono and diolefinic hydrocarbons; and

(b) cooling and partially condensing (6, 11) the dehydrogenation reactor effluent stream (10) and separating the dehydrogenation reactor effluent stream in a vapor-liquid separation zone (12) into a vapor phase process stream (13) which comprises hydrogen and a liquid phase process stream (14) which comprises dissolved hydrogen, light hydrocarbon(s), $C_4$-plus paraffinic hydrocarbon(s), and $C_4$-plus mono- and diolefinic hydrocarbons;

characterised by the steps of

(c) passing a hydrogen feed stream (15) and the liquid phase process stream (14) through a selective hydrogenation reaction zone (17) which contains a selective hydrogenation catalyst and is maintained at diolefin selective hydrogenation conditions and forming a selective hydrogenation reaction zone effluent stream (18) which contains $C_4$-plus mono-olefinic hydrocarbon(s) and is substantially free of $C_4$-plus diolefinic hydrocarbons; and

(d) passing the selective hydrogenation reaction zone effluent stream (18) into a stripping column (19) operated at conditions which result in the concentration of substantially all hydrogen and light hydrocarbon(s) present in the selective hydrogenation zone effluent stream into a stripping column overhead stream (20), and also producing a stripping column bottoms stream (21) which comprises $C_4$-plus paraffinic hydrocarbon(s) and $C_4$-plus monoolefinic hydrocarbon(s) and which is removed from the process as a product stream.

2. A process as claimed in claim 1, characterised in that the selective hydrogenation conditions include the presence of less than twice the stoichiometrically required amount of hydrogen for the selective hydrogenation of the diolefinic hydrocarbon(s) present in the liquid phase process stream to monoolefinic hydrocarbon(s).

3. A process as claimed in claim 1 or 2, characterised in that the mole ratio of hydrogen to diolefinic hydrocarbon(s) in the liquid phase process stream is maintained at from 1.0:1 to 1.8:1.

4. A process as claimed in any of claims 1 to 3, characterised in that a catalyst comprising nickel and sulfur and an inorganic refractory support material is employed within the selective catalytic hydrogenation zone.

5. A process as claimed in any of claims 1 to 3, characterised in that a catalyst comprising palladium and a refractory inorganic support material is employed within the selective catalytic hydrogenation zone.

6. A process as claimed in any of claims 1 to 5, characterised in that the product stream is passed into a separation zone in which olefinic hydrocarbons are concentrated into an olefin-rich stream which is withdrawn and a separation zone effluent stream which is rich in the paraffinic feed hydrocarbon and which contains minor amounts of olefinic hydrocarbon(s) is also formed, and in that at least a portion of said separation zone effluent stream is passed into the dehydrogenation zone as a recycle stream.

7. A process as claimed in claim 6, characterised in that the separation zone comprises a fixed bed of a solid adsorbent which selectively adsorbs either olefinic or paraffinic hydrocarbons.

8. A process as claimed in claim 6 or 7, characterised in that the recycle stream has a monoolefin concentration of from O.4 to l.9 mole percent.

9. A process as claimed in any of claims 1 to 5, characterised in that the feed stream comprises at least one $C_8$-plus paraffinic hydrocarbon, the product stream is passed into an alkylation zone (23) maintained at alkylation-promoting conditions which include the presence of an aromatic hydrocarbon and an alkylation catalyst, an alkylation zone effluent stream (24) which comprises an alkylaromatic hydrocarbon is produced and the alkylaromatic hydrocarbon is recovered (25, 26) from the alkylation zone effluent stream.

10. A process as claimed in claim 9, characterised in that the aromatic hydrocarbon is benzene.

11. A process as claimed in claim 9 or 10, characterised in that the alkylation zone effluent stream also comprises $C_8$-plus paraffinic hydrocarbon(s) and in that $C_8$-plus paraffinic hydrocarbon(s) are recovered from the alkylation zone effluent stream and recycled (3) into the dehydrogenation zone.

12. A process for the dehydrogenation of a paraffinic hydrocarbon which comprises the steps of:

(a) passing a paraffin feed stream (8) which comprises at least one $C_{10}$-plus linear paraffinic hydrocarbon through a dehydrogenation reaction zone (9) and forming a vapor phase dehydrogenation reaction zone effluent stream (10) which comprises a mixture of hydrogen, mono- and diolefinic $C_{10}$-plus linear hydrocarbons, and $C_{10}$-plus linear paraffin(s);

(b) separating hydrogen from the dehydrogenation reaction zone effluent stream (10) by partially condensing (6, 11) the dehydrogenation reaction zone effluent stream and separating the resultant two-phase admixture in a vapor-liquid separation zone (12) and forming a vapor phase stream (13) which is rich in hydrogen and a liquid phase process stream (14) comprising $C_{10}$-plus linear paraffin(s), hydrogen, and mono- and diolefinic $C_{10}$-plus linear hydrocarbons; characterised by the steps of

(c) passing the liquid phase process stream (14, 16) through a selective hydrogenation zone (17) maintained at diolefin selective hydrogenation conditions and in which the liquid phase process stream is contacted with a solid selective hydrogenation catalyst and thereby forming a hydrogenation zone effluent stream (18) which contains less than 0.4 mole percent $C_{10}$-plus diolefinic hydrocarbon(s);

(d) removing substantially all free hydrogen and $C_6$-minus hydrocarbon(s) from the hydrogenation zone effluent stream (18) by passing the hydrogenation zone effluent stream into a liquid ends stripping column (19), and producing a stripping column bottoms stream (21) which comprises a mixture of $C_{10}$-plus monoolefinic linear hydrocarbon(s) and $C_{10}$-plus linear paraffin(s);

(e) contacting the stripping column bottoms stream (21, 22) with an alkylation catalyst and with benzene within an alkylation zone (23) maintained at alkylation promoting conditions, and producing an alkylation zone effluent stream (24) which comprises $C_{10}$-plus linear paraffin(s) and alkylbenzene(s); and

(f) recovering the alkylbenzene(s) (26) from the alkylation zone effluent stream (24).

13. A process as claimed in any of claims 9 to 12, characterised in that the alkylation catalyst comprises HF.

**Patentansprüche**

1. Verfahren zur Dehydrierung eines paraffinischen Kohlenwasserstoffs, welches die Schritte umfasst, dass man

(a) einen Wasserstoff und wenigstens einen $C_4$-plus paraffinischen Einsatzkohlenwasserstoff enthaltenden Einsatzstrom (8) durch eine unter Dehydrierungsbedingungen gehaltenen Dehydrierungsreaktionszone (9) leitet und dadurch einen Wasserstoff, leichten Kohlenwasserstoff bzw. leichte Kohlenwasserstoffe mit weniger Kohlenstoffatomen pro Molekül als der Einsatzkohlenwasserstoff, $C_4$-plus paraffinischen Einsatzkohlenwasserstoff bzw. paraffinische Einsatz-

14

kohlenwasserstoffe und C$_4$-plus mono und diolefinische Kohlenwasserstoffe enthaltenden dampfphasischen Dehydrierungsreaktorproduktstrom (10) produziert, und

(b) den Dehydrierungsreaktorproduktstrom (10) kühlen und teilweise kondensieren (6,11) lässt und diesen in einer Dampf/Flüssigkeitstrennzone (12) in einen Wasserstoff enthaltenden Dampfphasenprozessstrom (13) und einen gelösten Wasserstoff, leichten Kohlenwasserstoff bzw. leichte Kohlenwasserstoffe, C$_4$-plus paraffinischen Kohlenwasserstoff bzw. paraffinische Kohlenwasserstoffe und C$_4$-plus mono- und diolefinische Kohlenwasserstoffe enthaltenden Flüssigphasenprozessstrom (14) abtrennt, durch die Schritte gekennzeichnet, dass man

(c) einen Wasserstoffeinsatzstrom (15) und den Flüssigphasenprozessstrom (14) durch eine, einen selektiven Hydrierungskatalysator enthaltende selektive Hydrierungsreaktionszone (17), in der selektive Hydrierungsbedingungen für Diolefine aufrechterhalten sind, leitet und einen C$_4$-plus monoolefinischen Kohlenwasserstoff bzw. monoolefinische Kohlenwasserstoffe enthaltenden und im wesentlichen von C$_4$-plus diolefinischen Kohlenwasserstoffen freien selektiven Hydrierungsreaktionszonenproduktstrom (18) bildet und

(d) den selektiven Hydrierungsreaktionszonenproduktstrom (18) in eine unter solchen Bedingungen betriebene Abstreiferkolonne (19) einleitet, die zur Konzentration des praktisch gesamten, im selektiven Hydrierungszonenproduktstrom vorhandenen Wasserstoffs und leichten Kohlenwasserstoffs bzw. der leichten Kohlenwasserstoffe in einem Abstreiferkolonnenkopfstrom (20) führen, und auch einen C$_4$-plus paraffinischen Kohlenwasserstoff bzw. paraffinische Kohlenwasserstoffe und C$_4$-plus monoolefinischen Kohlenwasserstoff bzw. monoolefinische Kohlenwasserstoffe enthaltenden Abstreiferkolonnensumpfstrom (21), der dem Verfahren als ein Produktstrom entzogen wird, produziert.

2. Verfahren nach Anspruch 1, dadurch gekennzeichnet, dass die selektiven Hydrierungsbedingungen die Gegenwart von weniger als der zweifachen stöchiometrisch zur selektiven Hydrierung des (der) im Flüssigphasenprozessstrom vorhandenen diolefinischen Kohlenwasserstoffs bzw. Kohlenwasserstoffe in monoolefinischen Kohlenwasserstoff bzw. monoolefinische Kohlenwasserstoffe erforderlichen Wasserstoffmenge umfassen.

3. Verfahren nach Anspruch 1 oder 2, dadurch gekennzeichnet, dass das Molverhältnis von Wasserstoff zu diolefinischem Kohlenwasserstoff bzw. diolefinischen Kohlenwasserstoffen in dem Flüssigphasenprozessstrom bei von 1,0:1 zu 1,8:1 beibehalten wird.

4. Verfahren nach einem der Ansprüche 1 bis 3 dadurch gekennzeichnet, dass ein Katalysator aus Nickel und Schwefel und einem anorganischen feuerfesten Trägermaterial innerhalb der selektiven katalytischen Hydrierungszone verwendet wird.

5. Verfahren nach einem der Ansprüche 1 bis 3, dadurch gekennzeichnet, dass ein Katalysator aus Palladium und einem feuerfesten anorganischen Trägermaterial innerhalb der selektiven katalytischen Hydrierungszone verwendet wird.

6. Verfahren nach einem der Ansprüche 1 bis 5, dadurch gekennzeichnet, dass der Produktstrom in eine Trennzone geleitet wird, in welcher olefinische Kohlenwasserstoffe in einen olefinreichen Strom, welcher entzogen wird, konzentriert werden, wobei auch ein Trennzonenproduktstrom, welcher an dem paraffinischen Einsatzkohlenwasserstoff reich ist und geringe Mengen an olefinischem Kohlenwasserstoff bzw. olefinischen Kohlenwasserstoffen erhält, gebildet wird und dass wenigstens ein Teil des besagten Trennzonenproduktstroms in die Dehydrierungszone als ein Rückführungsstrom geleitet wird.

7. Verfahren nach Anspruch 6, dadurch gekennzeichnet, dass die Trennzone ein Festbett aus einem festen Adsorptionsmittel enthält, welches entweder olefinische oder paraffinische Kohlenwasserstoffe selektiv adsorbiert.

8. Verfahren nach Anspruch 6 oder 7, dadurch gekennzeichnet, dass der Rückführungsstrom eine Monoolefinkonzentration von 0,4 bis 1,9 Molprozent aufweist.

9. Verfahren nach einem der Ansprüche 1 bis 5, dadurch gekennzeichnet, dass der Einsatzstrom aus wenigstens einem C$_8$-plus paraffinischen Kohlenwasserstoff besteht, der Produktstrom in eine Alkylierungszone (23) geleitet wird, in welcher alkylierungsfördernde Bedingungen, umfassend die Anwesenheit eines aromatischen Kohlenwasserstoffs und eines Alkylierungskatalysators aufrechterhalten werden, ein einen alkylaromatischen Kohlenwasserstoff enthaltender Alkylierungszonenproduktstrom (24) gebildet und der alkylaromatische Kohlenwasserstoff aus dem Alkylierungszonenproduktstrom wiedergewonnen (25 26) wird.

10. Verfahren nach Anspruch 9, dadurch gekennzeichnet, dass der aromatische Kohlenwasserstoff Benzol ist.

11. Verfahren nach Anspruch 9 oder 10 dadurch gekennzeichnet, dass der Alkylierungszonenproduktstrom auch C$_8$-plus paraffinischen Kohlenwasserstoff bzw. paraffinische Kohlenwasserstoffe enthält und dass C$_8$-plus paraffinischer Kohlenwasserstoff bzw. paraffinische Kohlenwasserstoffe aus dem Alkylierungszonenproduktstrom wiedergewonnen und in die Dehydrierungszone rückgeführt (3) werden.

12. Verfahren zur Dehydrierung eines paraffinischen Kohlenwasserstoffs, welches aus den Schritten besteht, dass man

(a) einen, wenigstens einen C$_{10}$-plus linearen paraffinischen Kohlenwasserstoff enthaltenden paraffinischen Einsatzstrom (8) durch eine Dehydrierungsreaktionszone (9) leitet und einen, ein Gemisch aus Wasserstoff, mono- und diolefinischen C$_{10}$-plus linearen Kohlenwasserstoffen und C$_{10}$-plus linearem Paraffin bzw. linearen Paraffinen enthaltenden dampfphasischen Dehydrierungsreaktionszonenproduktstrom (10) bildet,

(b) Wasserstoff aus dem Dehydrierungsreaktionszonenproduktstrom (10) durch teilweise Kondensation (6, 11) des Dehydrierungsreaktionszonenproduktstroms abtrennt, und die sich ergebende zweiphasige Beimischung in einer Dampf/Flüssigkeitstrennzone (12) abtrennt, und einen an Wasserstoff reichen Dampfphasenstrom (13) und einen $C_{10}$-plus lineares Paraffin bzw. lineare Paraffine, Wasserstoff und mono- und diolefinische $C_{10}$-plus lineare Kohlenwasserstoffe enthaltenden Flüssigphasenprozessstrom (14) bildet, gekennzeichnet durch die Schritte, dass man

(c) den Flüssigphasenprozessstrom (14, 16) durch eine selektive Hydrierungszone (17) leitet, in der selektive Hydrierungsbedingungen für Diolefine aufrechterhalten sind und in welcher der Flüssigphasenprozessstrom mit einem festen selektiven Hydrierungskatalysator in Berührung gebracht wird und man dadurch einen weniger als 0,4 Molprozent $C_{10}$-plus diolefinischen Kohlenwasserstoff bzw. diolefinische Kohlenwasserstoffe enthaltenden Hydrierungszonenproduktstrom (18) bildet,

(d) praktisch den gesamten freien Wasserstoff und $C_6$-minus Kohlenwasserstoff(e) aus dem Hydrierungszonenproduktstrom (18) durch Einleiten des Hydrierungszonenproduktstroms in eine Flüssigenden-Abstreiferkolonne (19) entfernt und einen aus einem Gemisch von $C_{10}$-plus monoolefinischem linearen Kohlenwasserstoff bzw. monoolefinischen linearen Kohlenwasserstoffen und $C_{10}$-plus linearem Paraffin bzw. linearen Paraffinen bestehenden Abstreiferkolonnensumpfstrom (21) bildet,

(e) den Abstreiferkolonnensumpfstrom (21, 22) mit einem Alkylierungskatalysator und mit Benzol innerhalb einer Alkylierungszone (23), in der alkylierungsfördernde Bedingungen aufrechterhalten sind, in Berührung bringt und einen aus $C_{10}$-plus linearem Paraffin bzw. linearen Paraffinen und Alkylbenzol(en) bestehenden Alkylierungszonenproduktstrom (24) bildet und

(f) das Alkylbenzol bzw. die Alkylbenzole (26) aus dem Alkylierungszonenproduktstrom (24) rückgewinnt.

13. Verfahren nach einem der Ansprüche 9 bis 12, dadurch gekennzeichnet, dass der Alkylierungskatalysator HF enthält.

**Revendications**

1. Un procédé de déshydrogénation d'un hydrocarbure paraffinique qui comprend les opérations qui consistent:

(a) à faire passer un courant de charge (8) comprenant de l'hydrogène et au moins un hydrocarbure paraffinique en $C_4+$ dans une zone de réaction de déshydrogénation (9) maintenue dans des conditions de déshydrogénation et produire ainsi un courant d'effluent (10) du réacteur de déshydrogénation en phase vapeur qui est composé d'hydrogène, d'hydrocarbure(s) léger(s) sous-produit(s) ayant moins d'atomes de carbone par molécule que l'hydrocarbure de charge, d'hydrocarbure(s) paraffinique(s) de charge en $C_4+$ et d'hydrocarbures mono- et dioléfiniques en $C_4+$,

(b) à refroidir et condenser partiellement (6, 11) le courant d'effluent (10) du réacteur de déshydrogénation et séparer le courant d'effluent du réacteur de déshydrogénation dans une zone (12) de séparation vapeur liquide en un courant (13) de production en phase vapeur qui contient de l'hydrogène et en un courant (14) de production en phase liquide qui est constitué d'hydrogène dissous, d'hydrocarbure(s) léger(s), d'hydrocarbure(s) paraffinique(s) en $C_4+$ et d'hydrocarbures mono- et dioléfin ques en $C_4+$,

(c) à faire passer un courant (15) de charge d'hydrogène et le courant (14) de production en phase liquide dans une zone (17) de réaction d'hydrogénation sélective qui contient un catalyseur d'hydrogénation sélective et est maintenue dans des conditions d'hydrogénation sélective des dioléfines, et former un courant d'effluent (18) de la zone de réaction d'hydrogénation sélective qui contient des hydrocarbure(s) mono-oléfinique(s) en $C_4+$ et est pratiquement exempt d'hydrocarbures dioléfiniques en $C_4+$, et

(d) à faire passer le courant d'effluent (18) de la zone de réaction d'hydrogénation sélective dans une colonne d'extraction (19) fonctionnant dans des conditions qui permettent la concentration de pratiquement la totalité de l'hydrogène et des hydrocarbure(s) léger(s) présent(s) dans le courant d'effluent de la zone d'hydrogénation dans un courant de tête (20) de la colonne d'extraction, et également produire un courant de queue (21) de la colonne d'extraction qui contient des hydrocarbure(s) paraffinique(s) en $C_4+$ et des hydrocarbure(s) monoléfinique(s) en $C_4+$ et qui est retiré du procédé comme courant de produit.

2. Un procédé suivant la revendication 1, caractérisé en ce que les conditions d'hydrogénation sélective comprennent la présence de moins de 2,0 fois la quantité stoechiométriquement requise d'hydrogène pour l'hydrogénation sélective des hydrocarbure(s) dioléfinique(s) présente dans le courant de production en phase liquide en des hydrocarbure(s) monoléfinique(s).

3. Un procédé suivant la revendication 1 ou 2, caractérisé en ce que le rapport molaire de l'hydrogène aux hydrocarbure(s) dioléfinique(s) dans le courant de production en phase liquide est maintenu entre 1,0:1,0 et 1,8:1,0.

4. Un procédé suivant l'une quelconque des revendications 1 à 3, caractérisé en ce que, dans la zone d'hydrogénation catalytique sélective, on utilise un catalyseur composé de nickel et de soufre et d'un matériau de support minéral réfractaire.

5. Un procédé suivant l'une quelconque des revendication 1 à 3, caractérisé en ce que, dans la zone d'hydrogénation catalytique sélective, on utilise un catalyseur constitué de palladium et d'un matériau de support minéral réfractaire.

6. Un procédé suivant l'une quelconque des revendications 1 à 5, caractérisé en ce que le courant de produit traverse une zone de séparation dans laquelle les hydrocarbures oléfiniques sont concentrés en un courant riche en oléfines qui est retiré, et il se forme également un courant d'effluent de zone de séparation qui est riche en l'hydrocarbure de charge paraffinique et qui contient des quantités mineures d'hydrocarbure(s) oléfinique(s) et en ce qu'au moins une portion du courant d'effluent de la zone de séparation traverse la zone de déshydrogénation comm courant de recyclage.

7. Un procédé suivant la revendication 6, caractérisé en ce que la zone de séparation comprend un lit fixe d'un adsorbant solide qui adsorbe sélectivement les hydrocarbures oléfiniques ou les hydrocarbures paraffiniques.

8. Un procédé suivant la revendication 6 ou 7, caractérisé en ce que le courant de recyclage a une teneur en mono-oléfines comprise entre 0,4 et 1,9 mole %.

9. Un procédé suivant l'une quelconque des revendications 1 à 5, caractérisé en ce que le courant de charge contient au moins un hydrocarbure paraffinique en $C_8+$ et le courant de produit est envoyé dans une zone d'alcoylation (23) maintenue dans des conditions favorisant l'alcoylation, qui comprennent la présence d'un hydrocarbure aromatique et d'un catalyseur d'alcoylation, et un courant d'effluent (24) de la zone d'alcoylation, qui contient un hydrocarbure alcoylaromatique, est produit et les hydrocarbures alcoylaromatiques du courant d'effluent de la zone d'alcoylation sont récupérés (25, 26).

10. Un procédé suivant la revendication 9, caractérisé en ce que l'hydrocarbure aromatique est le benzène.

11. Un procédé suivant la revendication 9 ou 10, caractérisé en ce que le courant d'effluent de la zone d'alcoylation contient également des hydrocarbure(s) paraffinique(s) en $C_8+$ et les hydrocarbure(s) paraffinique(s) en $C_8+$ sont récupérés à partir du courant d'effluent de la zone d'alcoylation et recyclés (3) dans la zone de déshydrogénation.

12. Un procédé de déshydrogénation d'un hydrocarbure paraffinique qui comprend les opérations qui consistent:

(a) à faire passer un courant (8) de charge de paraffines qui contient au moins un hydrocarbure paraffini- que linéaire en $C_{10}+$ dans une zone (9) de réaction de déshydrogénation et former un courant d'effluent (10) de la zone de réaction de déshydrogénation en phase vapeur, qui contient un mélange d'hydrogène, d'hydrocarbures linéaires mono- et dioléfiniques en $C_{10}+$ et des paraffines linéaires en $C_{10}+$,

(b) à séparer l'hydrogène du courant d'effluent (10) de la zone de réaction de déshydrogénation par une condensation partielle (6, 11) du courant d'effluent de la zone de réaction de déshydrogénation, et séparer le mélange en deux phases résultant dans une zone (12) de séparation vapeur-liquide et former un courant en phase vapeur riche en hydrogène et un courant (14) de production en phase liquide contenant des paraffine(s) linéaire(s) en $C_{10}+$, de l'hydrogène et des hydrocarbures linéaires mono- et dioléfiniques en $C_{10}+$, caractérisé en ce que:

(c) on fait passer le courant (14) de production en phase liquide dans une zone (17) d'hydrogénation sélective maintenue dans des conditions d'hydrogénation sélective des dioléfines et dans laquelle le courant de production en phase liquide est mis en contact avec un catalyseur solide d'hydrogénation sélective, formant ainsi un courant d'effluent (18) de zone d'hydrogénation qui contient moins de 0,4 mole % d'hydrocarbure(s) dioléfinique(s) en $C_{10}+$,

(d) on sépare pratiquement la totalité de l'hydrogène libre et des hydrocarbure(s) en $C_6-$ du courant d'effluent (18) de la zone d'hydrogénation en faisant passer le courant d'effluent de la zone d'hydrogénation dans une colonne (19) d'extraction des fractions légères, et on produit un courant de queue (21) de la colonne d'extraction qui contient un mélange d'hydrocarbure(s) linéaire(s) monooléfinique(s) en $C_{10}+$ et de paraffine(s) linéaire(s) en $C_{10}+$,

(e) on met en contact le courant de queue (21, 22) de la colonne d'extraction avec un catalyseur d'alcoylation et avec du benzène dans une zone d'alcoylation (23) maintenue dans des conditions favorisant l'alcoylation, et on produit un courant d'effluent (24) de la zone d'alcoylation qui contient des paraffine(s) linéaire(s) en $C_{10}+$ et des alcoylbenzène(s), et

(f) on récupère les alcoylbenzène(s) (26) à partir du courant d'effluent (24) de la zone d'alcoylation.

13. Un procédé suivant l'une quelconque des revendications 9 à 12, caractérisé en ce que le catalyseur d'alcoylation contient HF.

$C_{10}-C_{15}$ Paraffins

Heat Exchanger

Heater

Dehydrogenation Reactor

Selective Hydrogenation Reactor

$H_2$

Separator

Heat Exchanger

Stripper

$C_{10}-C_{15}$ Paraffins / Olefins

DRAWING I

DRAWING II

$C_{10}-C_{15}$ PARAFFINS

DEHYDROGENATION REACTOR

DRAWING III

SELECTIVE HYDROGENATION REACTOR

STRIPPER

ALKYLBENZENES

ALKYLATION ZONE

FRACTIONATION ZONE

$H_2$

BENZENE

0 158 503